# EUROPEAN PATENT SPECIFICATION

(11) **EP 1 617 878 B1**
(45) Date of publication and mention of the grant of the patent: **22.11.2006**
(21) Application number: 04713715.3
(22) Date of filing: 23.02.2004
(51) Int. Cl.: A61L 2/28

(54) **HIGH THROUGHPUT BIOLOGICAL INDICATOR READER**
LESEGERÄT FÜR BIOLOGISCHE INDIKATOREN MIT HOHEM DURCHSATZ
LECTEUR D'INDICATEURS BIOLOGIQUES A RENDEMENT ELEVE

(30) Priority: 01.04.2003 US 405943
(43) Date of publication of application: 25.01.2006
(73) Proprietor: 3M Innovative Properties Company, St. Paul, MN 55133-3427 (US)
(72) Inventor: BEHUN, Bryan, S., Saint Paul, MN 55133-3427 (US); FLASKEY, Randy, L., Saint Paul, MN 55133-3427 (US)
(74) Representative: Meyers, Hans-Wilhelm
(86) International application number: PCT/US2004/005210
(87) International publication number: WO 2004/093925

(56) References cited:
- US-A- 6 025 189
- US-B1- 6 485 979

## Description

The invention relates to techniques for determining the efficacy of a sterilization cycle and, more particularly, techniques for reading fluorescence from a biological sterilization indicator to determine the efficacy of a sterilization cycle.

### BACKGROUND

Sterilization generally refers to the process of eliminating all bacteria and other living organisms from the surfaces of instruments, medical devices, implants and other articles used in sterile surgical procedures. A conventional thermal sterilization process uses steam under pressure. Low-temperature chemical sterilization processes use ethylene oxide, hydrogen peroxide, hydrogen peroxide/plasma, or peracetic acid in liquid or vapor form as the sterilant, as well as gamma irradiation and electron beam sterilization.

In each sterilization cycle, the sterilizer is designed to kill all viable living organisms within a sterilization chamber. To achieve this objective, health care personnel must select the appropriate sterilization process, carefully monitor its parameters and employ adequate preparation, packaging and sterilizer loading techniques.

To verify the efficacy of a sterilization cycle, health care facilities and surgical equipment manufacturers typically use sterilization process monitors. A sterilization process monitor is a device that indicates whether a sterilization cycle met the required conditions necessary to achieve sterilization. Examples of sterilization process monitors include: biological indicators, chemical indicators, chemical integrators, Bowie-Dick test packs, and challenge packs.

A biological indicator, in particular, carries a biological agent, and indicates successful sterilization when the biological agent has been killed. The biological indicator is typically placed in a test package within a load containing articles to be sterilized. The biological indicator indicates successful sterilization when the biological agent has been kil led. The biological agent carried by the biological indicator typically is a test organism which is many times more resistant to the sterilization process than most organisms which would be present by natural contamination.

After completion of the sterilization cycle, the biological indicator is incubated to determine whether any of the test organisms survived the sterilization procedure. If incubation produces growth in the biological indicator, the sterilization cycle was not efficacious. Accordingly, detection of biological indicator growth serves as an indication that devices subjected to the sterilization cycle may not be sterile, i.e., that the sterilization process failed.

Examples of existing biological indicators are the 3M^{™} Attest^{™} Rapid Readout Biological Indicators available from 3M Company of St. Paul, Minnesota. Different versions of the Attest Rapid Readout Biological Indicators are designed to determine the efficacy of steam and ethylene oxide sterilization processes. The Attest Rapid Readout Biological Indicators include a flexible polypropylene vial with a spore strip containing viable population of *Bacillus stearothermophilus* or *Bacillus subtilis* spores. The vial also contains a growth medium which is a modified tryptic soy broth contained in a crushable glass ampule.

In the Attest Rapid Readout Biological Indicators, the presence of a spore-associated enzyme indicates spore growth in the biological indicator. The presence of the active enzyme produces a detectable fluorescence. If the sterilization cycle is not efficacious, both the spore and the enzyme remain active, resulting in a fluorescent product. Following an incubation period, a reader determines whether the vial produces fluorescence, and thereby determines the efficacy of the sterilization cycle. Ordinarily, the reader and incubator are integrated, such that the biological indicators must be incubated before the reader can be activated to read the biological indicators.

### SUMMARY

In general, the invention is directed to a high throughput reader for reading biological indicators to detect fluorescence and thereby determine the efficacy of a sterilization cycle. The reader permits a large number of biological indicators to be read in rapid succession. For example, the biological indicators may be pre-incubated in a separate, high capacity incubator and then placed in a high capacity reader for immediate fluorescence detection. Notably, the reader makes a positive or negative growth determination based on a single fluorescence threshold comparison for each pre-incubated biological indicator, rather than a series of measurements taken during the course of incubation.

The single fluorescence threshold comparison involves comparison of measured fluorescence to a threshold fluorescence. However, the threshold fluorescence is combined with a baseline fluorescence measured from a reference biological indicator placed in an applicable measurement well within the reader. The reader initially acquires the baseline fluorescence from the reference biological indicator, and thereafter uses the baseline fluorescence with the threshold fluorescence for subsequent biological indicators processed by the reader for more accurate detection. In addition, the reader may generate output for storage, processing and display of biological indicator results.

In one embodiment, the invention provides a method comprising obtaining baseline fluorescence measurements for reference biological indicators placed within a plurality of measurement wells, receiving pre-incubated biological indicators within the measurement wells, obtaining a single fluorescence emission measurement for each of the pre-incubated biological indicators, and comparing the single fluorescence emission measurements obtained for each of the pre-incubated biological indicators to threshold fluorescence values for the respective measurement well to detect biological growth in the pre-incubated biological indicators, wherein the comparison takes into account the baseline fluorescence measurements for the respective measurement wells.

In another embodiment, the invention provides a device comprising a fluorescence emission reader to measurement fluorescence measurements for biological indicators placed within a plurality of measurement wells, and a processor to control the fluorescence emission reader to obtain baseline fluorescence measurements for each of a plurality of reference biological indicators placed within the measurement wells, and obtain a single fluorescence emission measurement for each of a plurality of pre-incubated biological indicators placed within the measurement wells, wherein the processor compares the single fluorescence emission measurements obtained for each of the pre-incubated biological indicators to the threshold fluorescence values for the respective measurement well to detect biological growth in the pre-incubated biological indicators, wherein the comparison takes into account the baseline fluorescence measurements for the respective measurement wells.

In a further embodiment, the invention provides a computer-readable medium comprising instructions to cause a processor to obtain baseline fluorescence measurements for reference biological indicators placed within a plurality of measurement wells, obtain a single fluorescence emission measurement for each of a plurality of pre-incubated biological indicators placed within the measurement wells, and compare the single fluorescence emission measurements obtained for each of the pre-incubated biological indicators to threshold fluorescence values for the respective measurement well to detect biological growth in the pre-incubated biological indicators, wherein the comparison takes into account the baseline fluorescence measurements for the respective measurement wells.

In an added embodiment, the invention provides a method comprising obtaining a single fluorescence measurement for each of a plurality of pre-incubated biological indicators placed in measurement wells, and comparing the single fluorescence emission measurements to threshold fluorescence values for the respective measurement wells in which the pre-incubated biological indicators are placed, wherein the comparison takes into account baseline fluorescence measurements obtained for reference biological indicators placed in the respective measurement wells.

In a further embodiment, the invention provides a device comprising a fluorescence emission reader to obtain fluorescence measurements for biological indicators placed within a plurality of measurement wells, and a processor to control the fluorescence reader to obtain a single fluorescence measurement for each of a plurality of pre-incubated biological indicators placed in the measurement wells, wherein the processor compares the single fluorescence emission measurements to threshold fluorescence values for the respective measurement wells in which the pre-incubated biological indicators are placed, wherein the comparison takes into account baseline fluorescence measurements obtained for reference biological indicators placed in the respective measurement wells.

The invention may provide a number of advantages. For example, the biological indicator reader described herein promotes high capacity and high throughput. The reader accommodates a plurality of pre-incubated biological indicators, and evaluates each of the biological indicators using a single fluorescence measurement. The biological indicators are exposed to a sterilization cycle and then pre-incubated in a separate, high capacity incubator prior to placement in the measurement wells associated with the reader.

The reader obtains initial baseline measurements for each measurement well using a single set of reference biological indicators. The reader then uses the baseline measurements to establish a detection threshold in combination with a threshold fluorescence for each measurement well, and uses the detection threshold for rapid, single-measurement evaluation of multiple sets of pre-incubated biological indicators. In this manner, the reader promotes detection accuracy for each measurement well.

Pre-incubation of the biological indicators permits omission of incubators within the reader, making the reader more compact and less complex, and conserving lab space. In addition, pre-incubation in a separate incubator permits immediate evaluation of the pre-incubated biological indicators by the reader. The ability to evaluate the pre-incubated biological indicators with a single measurement increases throughput. As a further advantage, the reader may be configured to provide output to a data logging software application, enabling convenient storage and review of the results obtained by the reader.

The details of one or more embodiments of the invention are set forth in the accompanying drawings and the description below. Other features, objects, and advantages of the invention will be apparent from the description and drawings, and from the claims.

### BRIEF DESCRIPTION OF DRAWINGS

FIG. 1 is a block diagram illustrating a system for analysis of biological indicators.
FIG. 2 is a side perspective view of an exemplary biological indicator reader.
FIG. 3 is a top perspective view of an exemplary incubator.
FIG. 4 is a block diagram illustrating a biological indicator reader.
FIG. 5 is a flow diagram illustrating an exemplary method for reading biological ind icators.
FIG. 6 is a flow diagram further illustrating an exemplary method for reading biological indicators.
FIG. 7 is a diagram of an exemplary user interface screen presenting results produced by a biological indicator reader.
FIG. 8 is a diagram of an exemplary data log containing results produced by a biological indicator reader.

### DETAILED DESCRIPTION

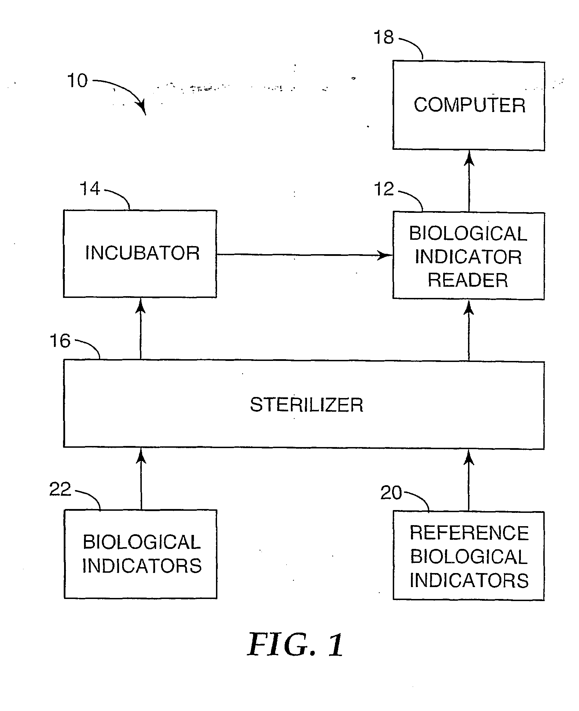

FIG. 1 is a block diagram illustrating a system 10 for analysis of biological indicators. As shown in FIG. 1, system 10 may include a biological indicator reader 12, a biological indicator incubator 14, a sterilizer 16 and a computer 18. Reader 12 processes reference biological indicators 20 to obtain initial baseline fluorescence measurements for subsequent evaluation of biological indicators 22 to detect proper sterilization. In general, system 10 promotes high capacity and high throughput in the reading of biological indicators 22 to detect fluorescence and thereby determine the efficacy of a sterilization cycle.

System 10 may be used within a health care facility to monitor sterilization efficacy for a large number of sterilization loads. Alternatively, system 10 may be especially useful in evaluating sterilization efficacy for equipment, instruments, medical devices, implants and other articles prior to commercial release of such articles from a manufacturing site. Accordingly, system 10 may be useful within the industrial and original equipment manufacturer (OEM) sterilization markets where large volumes are involved.

As a further alternative, system 10 may be used for commercial release of biological indicators 22 themselves. Specifically, samples from a manufactured lot of biological indicators 22 may be analyzed using system 10 to verify proper operation or manufacture of the biological indicators before they are commercially released, i.e., for manufacturing release testing. Thus, system 10 may be particularly useful for health care facilities, OEMs or sterilization service providers, as well as biological indicator manufacturers. In each case, processing of large volumes of medical devices or biological indicators is involved, making high throughput desirable.

In operation, biological indicator reader 12 obtains baseline fluorescence emission measurements for reference biological indicators 20 placed within a plurality of measurement wells in the reader. Sterilizer 16 exposes reference biological indicators 20 to a sterilization cycle before the reference biological indicators are placed in biological indicator reader 12. Sterilizer 16 also exposes groups of biological indicators 22 to a sterilization cycle. Then, incubator 14 pre-incubates biological indicators 22 before they are placed within measurement wells of reader 12.

Reader 12 obtains a single fluorescence emission measurement for each of the pre-incubated biological indicators 22, and compares the single fluorescence emission measurements obtained for each of the pre-incubated biological indicators to the sum of a predetermined threshold fluorescence and the initial baseline fluorescence measurement obtained for the respective measurement well using reference biological indicators 20. Alternatively, the baseline fluorescence measurement can be subtracted from the single fluorescence measurement, and the result compared to the predetermined threshold fluorescence. In either case, the predetermined threshold fluorescence may be determined as a function of the amount of fluorescence determined to be indicative of biological growth within a biological indicator.

As will be described, the comparison enables reader 12 to detect biological growth in pre-incubated biological indicators 22. In particular, if the single fluorescence emission measurement obtained for a biological indicator 22 in a given measurement well exceeds the sum of the threshold fluorescence and the baseline fluorescence measurement for that well, reader 12 indicates detection of growth and hence failure of the sterilization cycle.

Reader 12 may communicate the indication and other information to computer 18 for presentation to a user or storage in a data log for later review. Computer 18 may take the form of a personal computer, computer workstation, or other computing device. Reader 12 may communicate with computer 18 via a serial interface, network interface, or the like, to transfer reader information. Computer 18 provides a user interface, such as a display device and user input media. In addition, computer 18 executes a software application that drives collection, processing and presentation of information received from reader 12. In particular, computer 18 presents information obtained from reader 12 on a display device and logs the information to a data log file.

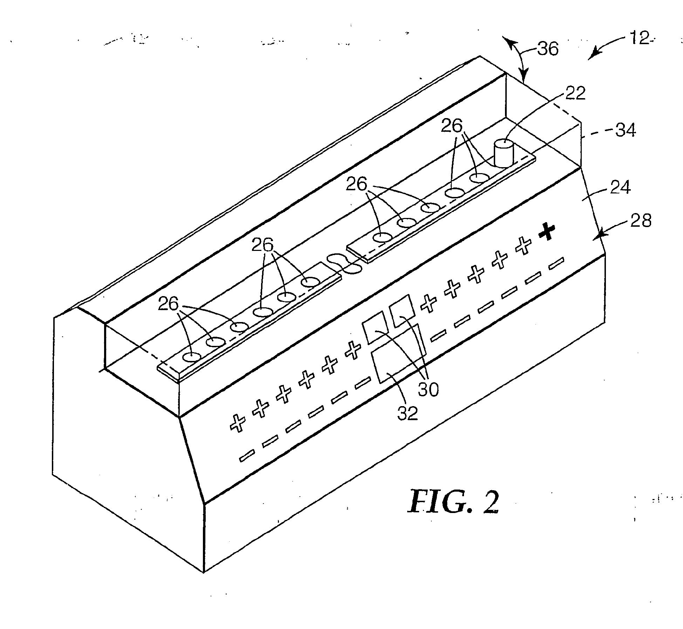

FIG. 2 is a perspective view of one embodiment of a biological indicator reader 12. As shown in FIG. 2, reader 12 includes a housing 24, which provides a plurality of measurement wells 26. Each measurement well 26 includes an associated display on a display panel 28. Display panel 28 may include "plus" and "minus" symbols that are selectively backlit to indicate the pertinent result for the biological indicator in the measurement well 26 aligned with the symbols. In addition, operator interaction with reader 12 may be accomplished with optional scroll buttons 30 and operator display 32. FIG. 2 also shows a pre-incubated biological indicator 22 residing in one of measurement wells 26. Reader 12 further includes a cover 34, which is pivotable generally along an arc 36 to alternately expose and cover measurement wells 26.

Measurement wells 26 may be arranged in groups or individually designated to receive particular types of biological indicators. In this manner, reader 12 may simultaneously process different types of biological indicators 22. To facilitate processing of different types of biological indicators 22, reader 12 may be configured to maintain different fluorescence thresholds for individual measurement wells 26. The fluorescence thresholds may be stored in memory and associated with particular measurement wells 26, and represent threshold values appropriate for the respective types of biological indicators 22 placed in the particular measurement wells 26.

In addition, reader 12 may store initial baseline fluorescence measurements for each measurement well 26. Reader 12 obtains the baseline fluorescence measurements by obtaining fluorescence measurements from reference biological indicators 20 placed within measurement wells 26. Reference biological indicators 20 may be placed within measurement wells 26, for example, upon periodic startup or initialization of reader 12. Each reference biological indicator 20 placed in a respective measurement well 26 should conform to the type of biological indicator 22 that will be subsequently evaluated in that measurement well in the course of operation of reader 12.

In this manner, reader 12 obtains, for each measurement well 26, a predetermined fluorescence threshold for the type of pre-incubated biological indicator 22 to be placed in the well and an initial baseline fluorescence measurement from the same type of reference biological indicator 20 placed in the well. The predetermined fluorescence threshold may be determined empirically or experimentally and loaded into memory associated with reader 12 at the factory or upon installation. Each reference biological indicator 20 is subjected to a sterilization cycle by sterilizer 16 prior to placement within the appropriate measurement well 26, but is not incubated. Consequently, each reference biological indicator 20 serves as a negative control (kill cycle) biological indicator of the same type that is desired to be read later for a sterilization cycle result.

Reader 12, in one embodiment, compares the fluorescence measurement obtained from each pre-incubated biological indicator 22 to the sum of the predetermined fluorescence threshold and the initial baseline fluorescence measurement for the respective measurement well 26. The summed value serves to fine-tune the predetermined fluorescence value for each measurement well by, in effect, discounting the baseline fluorescence value typically produced by a biological indicator that does not present biological growth. Again, alternative approach involves subtraction of the baseline fluorescence measurement from the fluorescence measurements obtained for pre-incubated biological indicators 22. Because pre-incubated biological indicators 22 are already incubated prior to placement within measurement wells 26, reader 12 is able to detect growth, by measurement of fluorescent emission, in a single measurement and comparison.

In some embodiments, biological indicators 20, 22 may be biological sterility indicators commercially available from 3M Company of Saint Paul, Minn., under the tradename 3M ATTEST, models 1291, 1292, 1292E, or 1294. In this case, each biological indicator 20, 22 includes a cap, a vial, and various contents. Biological indicators 20, 22 evidence the presence of a viable microorganism (such as spores) by the production of fluorescence within the vial after incubation in incubator 14.

Fluorescence emission may be accomplished by using a non-fluorescent substrate (such as 4-methylumbelliferyl-alpha-D-glucoside) in the vial and converting that non-fluorescent substrate to a fluorescent product by spore-associated enzyme activity. The spore-associated enzyme is preferably alpha-D-glucoside, which is one of the enzymes involved in the growth of the spore within the vial.

In operation, the operator of reader 12 first retrieves a reference biological indicator 20, which has been subjected to a sterilization cycle by sterilizer 18. The operator then crushes an ampule (not shown) in the reference biological indicator 20 and places the reference biological indicator into an appropriate measurement well 26. The operator can then direct reader 12 to read a desired measurement well 26, e.g., using scroll buttons 30 and observing display 32, to obtain a baseline fluorescence measurement. The operator then closes cover 34 and waits for reader 12 to obtain the baseline measurement the reference biological indicators 20. Alternatively, reader 12 may automatically detect the presence of a biological indicator 20, 22 in any given well and automatically read the biological indicator after insertion.

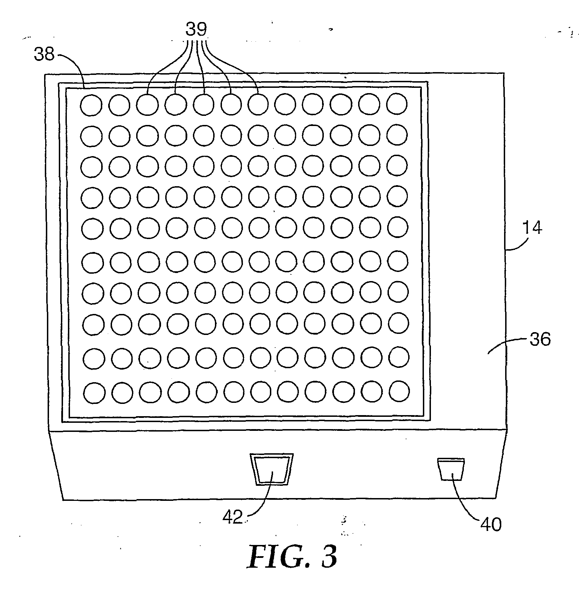

FIG. 3 is a front perspective view of an exemplary incubator 14. As shown in FIG. 3, incubator 14 includes a housing 36 defining an incubation bay 38 with multiple incubation wells 39 to receive and heat biological indicators 22 for incubation. One of more controls, such as a button 40, may be provided to control operation of incubator 14. In addition, incubator 14 may include a display panel 42 to present incubation information such as temperature or incubation time.

Incubator 14 supports simultaneous incubation of a large number of biological indicators 22. In the example of FIG. 3, incubator 14 is designed to accommodate and simultaneously incubate 120 biological indicators 22. Upon completion of the incubation cycle, pre-incubated biological indicators 22 can be transported, several at a time, to biological indicator reader 12 for fluorescence measurement and growth detection. As an example, reader 12 and incubator 14 may be configured so that twelve biological indicators can be transferred to the reader at one time. Hence, system 10 (FIG. 1) facilitates high throughput incubation and simultaneous reading of numerous biological indicators 22.

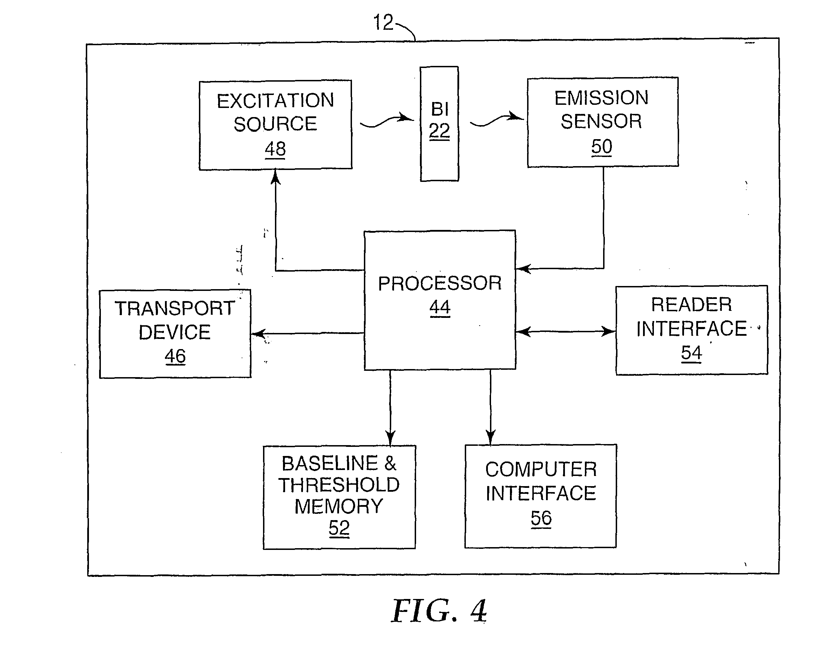

FIG. 4 is a block diagram illustrating biological indicator reader 12 in greater detail. As shown in FIG. 4, reader 12 includes a processor 44, a transport device 46, an excitation source 48, an emission sensor 50, a threshold and baseline memory 52, device interface 54 and computer interface 56. In general, processor 44 controls various operations within reader 12. For example, processor 44 drives transport device 46 to move excitation source 48 and emission sensor 50 between different measurement wells 26 within reader 12. In other words, reader 12 may include a single excitation source 48 and emission sensor 50 that travel together on a carriage between different measurement wells 26. The mechanical structure of reader 12, including transport device 46, may generally correspond to the structure of the reader described in commonly assigned U.S. Patent No. 6,025,189, to Bolea et al., with the exception that reader 12 may omit an incubator. In other embodiments, reader 12 may include multiple excitation sources and multiple emission sensors to permit rapid reading of larger numbers of biological indicators. In the example of FIG. 2, reader 12 includes twelve measurement wells. However, a smaller or larger number of measurement wells can be provided in other embodiments. Accordingly, the use of multiple excitation sources and emission sensors may be desirable in some instances

Processor 44 also controls excitation source 48 to transmit excitation energy to a pre-incubated biological indicator 22 placed within a measurement well 26 to cause the contents of the biological indicator to fluoresce. Emission sensor 50 senses fluorescent emission from each pre-incubated biological indicator 22 and transmits associated measurement information to processor 44. Notably, excitation source 48 and emission sensor 50 are also used to obtain baseline fluorescence measurements from reference biological indicators 20, e.g., upon startup or initialization of reader 12.

Excitation source 48 and emission sensor 50 may conform substantially to those described in the above-referenced patent to Bolea et al. For example, excitation source 48 may include a fluorescent lamp or flash tube source and a filter to pass an appropriate band of radiation. The light that is passed through the filter impinges on biological indicator 22 to excite the fluorescent material in the biological indicator. Processor 44 may be configured to selectively control the emission output of excitation source 48 as appropriate to excite different types of biological indicators that may be placed within reader 12 at a given time.

The fluorescence exhibited by biological indicator 22 can be collected by an integration cavity within the measurement well 26 in which the biological indicator is placed. The integration cavity collects the fluorescence emitted from biological indicator 22 and directs that fluorescence to emission sensor 50. As described in the above-referenced patent to Bolea et al., emission sensor 50 may include a filter and optical sensor configured to acquire a desired range of emission wavelengths indicative of fluorescence from biological indicator 22. Emission sensor 50 generates a fluorescence measurement value based on the received fluorescence and provides the value to processor 44. Emission sensor 50 may be configured to generate the fluorescence measurement value as a digital value or an analog signal that is converted to digital form before presentation to processor 44.

Upon receipt of baseline fluorescence measurements for reference biological indicators 20, processor 44 stores the measurements in threshold and baseline memory 52 and associates the measurements with the appropriate measurement wells 26, and hence the appropriate types of biological indicators that will be placed in the respective wells. During operation, upon receipt of fluorescence measurements for pre-incubated biological indicators 22, processor 44 retrieves the pertinent threshold fluorescence value and baseline fluorescence value from memory 52 and compares the sum of those values to the fluorescence measurement. Alternatively, processor 44 may retrieve a pre-calculated sum of the threshold fluorescence value and baseline fluorescence value from memory 44. As a further alternative, processor 44 may subtract the pertinent baseline fluorescence values from the fluorescence measurements for the pre-incubated biological indicators 22.

If the fluorescence measurement for the pre-incubated biological indicator 22 exceeds the sum of the threshold fluorescence value and baseline fluorescence value; processor 44 detects growth within the pre-incubated biological indicator and indicates failure of the sterilization cycle. In a sense, the sum may be considered a modified threshold value for the pertinent measurement well 26 and type of biological indicator. Processor 44 may present the failure indication, or lack thereof, via device interface 54.

Device interface 54 may include display panel 28 (FIG. 2). For example, the "plus" and "minus" symbols on display panel 28 may be selectively backlit to indicate the pertinent result for the biological indicator in the measurement well 26 aligned with the symbols. For example, lighting of the plus symbol indicates growth, and lighting of the minus symbol indicates lack of growth.

Processor 44 may take the form of a microprocessor, a microcontroller, a DSP, an ASIC, an FPGA, discrete logic circuitry, or the like. In addition to threshold and baseline memory 52, processor 44 access a computer-readable medium to obtain and execute instructions. Hence, in some embodiments, the invention may be directed to a computer-readable medium comprising instructions to cause processor 44 to carry out various functions as described herein. The computer-readable medium carrying the instructions may include any volatile, non-volatile, magnetic, optical, or electrical media, such as a RAM, ROM, CD-ROM, hard disk, removable magnetic disk, memory cards or sticks, NVRAM, EEPROM, flash memory, and the like, as well as propagating media such as electrical or optical communication links.

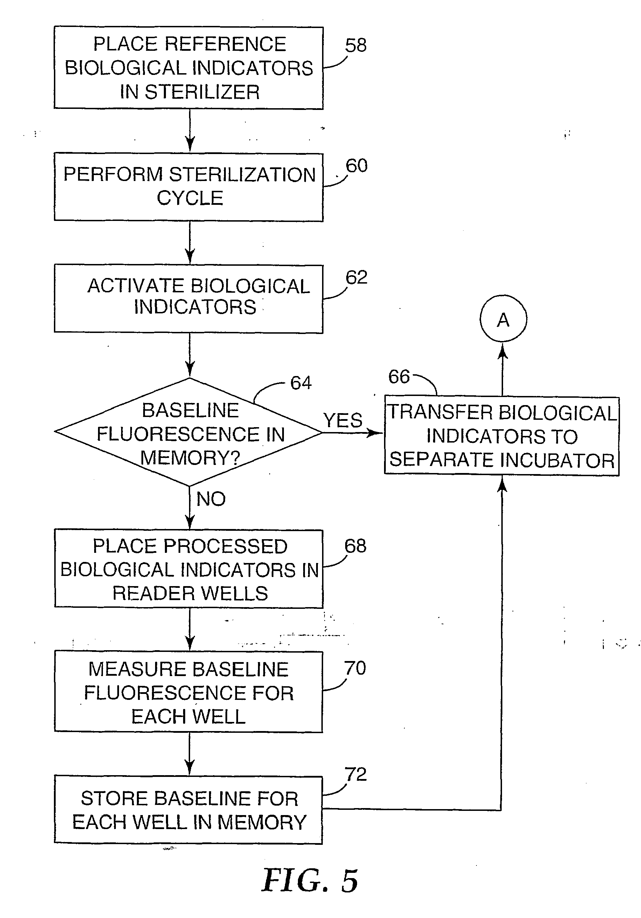

FIG. 5 is a flow diagram illustrating an exemplary method for reading biological indicators. As shown in FIG. 5, an operator places biological indicators 20 or 22 within sterilizer 16 (58), and performs a sterilization cycle (60). The sterilization cycle may include steam sterilization, dry heat sterilization, or chemical or radiation sterilization techniques. Different time frames, temperature regimes and sterilization cycles require the use of different types of biological indicators, as is known.

Following sterilization, the operator activates the biological indicators (62) to condition them for growth. In particular, in an embodiment in which biological indicators 20, 22 are implemented in a manner similar to the 3M ATTEST model 1291 biological indicator, the glass ampule containing a growth medium is crushed and its contents are applied to a dry strip containing the spores. If reader 12 already includes baseline fluorescence measurements in memory (64), the operator transfers the biological indicators to the incubator 14 separate from reader 12 (66).

If reader 12 does not already include baseline fluorescence measurements in memory, however, the biological indicators serve as the initial biological indicators to obtain the baseline measurements. In this case, the biological indicators subjected to the sterilization cycle are not placed in the incubator. Instead, the operator places the sterile-processed biological indicators into the measurement wells in reader 12 (68) to obtain the baseline measurements. Reader 12 measures the baseline measurements for each well (70) and stores the baseline measurements in memory (72).

The biological indicators used to obtain the baseline measurements may be sacrificial in the sense that they are merely discarded after the baseline measurements are obtained. Alternatively, they may be incubated (66) to detect growth and thereby verify the efficacy of the sterilization cycle. In either case, the biological indicators used to obtain the baseline measurements serve as reference biological indicators for the reading of biological indicators later evaluated by reader 12.

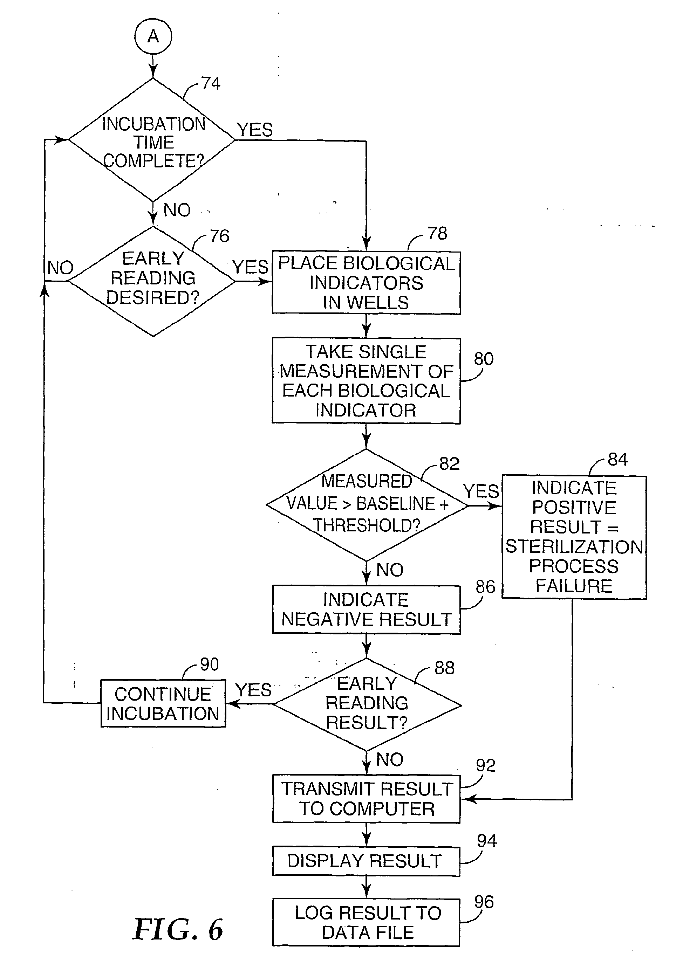

FIG. 6 is a flow diagram further illustrating an exemplary method for reading biological indicators. The process depicted in FIG. 6 continues from point A in FIG. 5. Following transfer of the biological indicators to the incubator (74), the operator determines whether the incubation time is complete (74). If the incubation time is not yet complete, the operator may determine whether an early reading is nevertheless desired (76). Upon completion of the incubation time, or for purposes of early reading, the operator places the pre-incubated (or partially incubated) biological indicators 22 in pertinent measurement wells 26 in reader 12 (78).

Reader 12 takes a single fluorescence measurement for each biological indicator 12 (80), and compares the measurement to the sum of the baseline measurement and the threshold fluorescence stored in memory 52 (82). If the measured value exceeds the sum of the baseline value and the predetermined threshold value (82), reader 12 indicates a positive result, i.e., detectable growth and therefore a sterilization process failure (84). If the measured value does not exceed the sum of the baseline value and the predetermined threshold value (82), reader 12 indicates a negative result (86).

If biological indicator 22 was used to obtain an early reading result (88), the operator may return the biological indicator to incubator 14 to continue incubation (90). If the reading was not an early reading result (88), or following indication of a positive result (84), reader 12 transmits information by routing the result to computer 18 (FIG. 1) (92). Computer 18 then displays the result for review by the operator (94), and logs the result to a data file (96) for archival to facilitate later analysis or verification of results. In some embodiments, computer 18 may receive results from multiple readers 12, e.g., by network communication, and process, log and display those results separately or together. The process depicted in FIGS. 5 and 6 may be repeated for each of the pre-incubated biological indicators 22 placed within measurement wells 26 of reader 12, and for subsequent sets of pre-incubated biological indicators loaded into the reader.

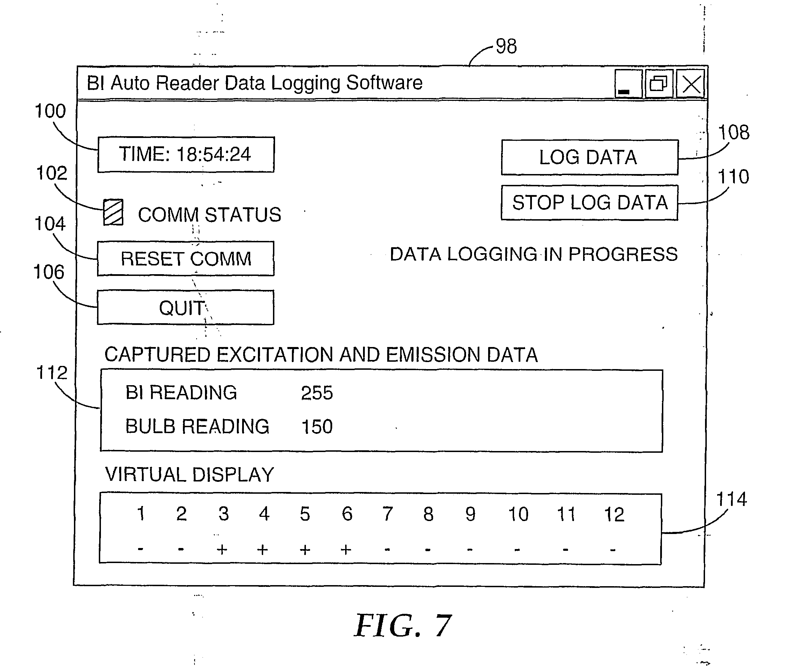

FIG. 7 is a diagram of an exemplary user interface screen 98 presenting results produced by a biological indicator reader 12 as described herein. User interface screen 98 may be presented to an operator by computer 22 (FIG. 1). User interface screen 98 may be generated by a data logging software application executed by computer 22. As shown in FIG. 7, user interface screen 98 may include a time field 100, and optionally a date field (not shown).

In addition, user interface screen 98 may include a communication status indicator 102 to indicate whether a communication link between computer 22 and reader 12 is active. User interface screen 98 also may include active buttons to reset communication (104), quit the application (106), log data obtained from reader 12 to a data file (108), and stop the logging of data (110).

User interface screen 98 also displays captured excitation and emission data (112) for individual biological indicators. In the example of FIG. 7, user interface screen 98 indicates a biological indicator (BI) reading of 255, e.g., on an 8-bit relative scale, and a bulb reading of 150 on the same scale. Reader 12 compares the biological indicator reading, i.e., the fluorescence measurement value, to the sum of the threshold fluorescence value and the based fluorescence value to detect growth. The bulb reading is a measure of the excitation source intensity. In various embodiments, the "bulb" may be a lamp of flash tube. The bulb reading enables the operator of reader 12 to verify that the excitation source is in working order. Reader 12 may automatically check the excitation source intensity to ensure it is above a predetermined failure intensity. In addition, reader 12 may automatically display an error if the value is below the failure intensity. The bulb reading displayed by user interface screen 98 can be monitored by the operator to anticipate diminishing bulb intensity and the need for preventative maintenance before the failure occurs.

Further, user interface screen 98 may include a virtual display 114 of the results obtained for each biological indicator 22 processed by reader 12. In the example of FIG. 7, virtual display 114 shows twelve different results for twelve different biological indicators 22. However, reader 12 may be modified to process any number of biological indicators 22, including much larger numbers of biological indicators, e.g., up to fifty or one-hundred biological indicators. The results may be represented as a "plus" sign to indicate a positive result, or a "negative" or "minus" sign to indicate a negative result. Thus, virtual display 114 may generally depict display panel 28 of reader 12. If the measurement value for a particular biological indicator exceeds the sum of the threshold value and the baseline value, user interface 98 shows a "plus" sign for the applicable biological indicator. As an alternative, or in addition, the actual measurement value for each biological indicator may be presented within virtual display 114.

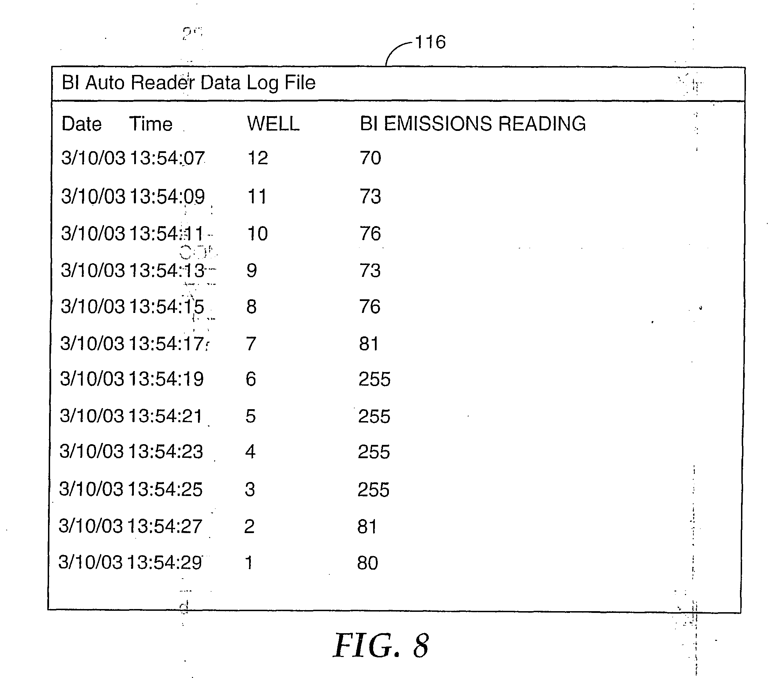

FIG. 8 is a diagram of an exemplary data log file 116 containing results produced by biological indicator reader 12. In the example of FIG. 8, data log file 116 may present time, date, well number, and emission reading information for a set of biological indicators 12 processed by reader 12. Thus, in addition to presenting real-time data via user interface screen 98, the data log application running on computer 22 may log data, including more detailed data, to a data log file for archival and subsequent analysis or verification by operators. In some cases, the data log file may serve as validation of the successful sterilization of instruments, medical devices, implants and other articles released for use in sterile surgical procedures. Alternatively, the data log file may serve as validation of the proper operation or manufacture of lots of biological indicators to be released for use in sterilization procedures.

System 10, as described herein, may offer a number of advantages. In particular, system 10 facilitates high capacity and high throughput reading of biological indicators for sterilization efficacy monitoring or manufacture release testing. Reader 12 is capable of detecting growth with a single measurement of a pre-incubated biological indicator. In addition, reader 12 can accommodate several biological indicators at the same time. The use of reference biological indicators to generate baseline fluorescence values for the measurement wells in reader 12 supports the ability to detect growth with a single measurement.

In addition, pre-incubation of the biological indicators permits omission of incubators within reader 12, and can make the reader more compact and less complex, and conserve laboratory space. As a further advantage, pre-incubation in a separate incubator permits immediate evaluation of the pre-incubated biological indicators by the reader 12, instead of waiting for incubation to proceed. This feature increases throughput in reader 12. The incorporation of a data logging software application, as described herein, enable convenient storage and review of the high number of results obtained by reader 12.

Various embodiments of the invention have been described. However, one skilled in the art will appreciate that various modifications may be made to these embodiments without departing from the scope of the invention. These and other embodiments are within the scope of the following claims.

## Claims

1. A method comprising:
obtaining a single fluorescence measurement for each of a plurality of pre-incubated biological indicators placed in measurement wells; and
comparing the single fluorescence emission measurements to threshold fluorescence values for the respective measurement wells in which the pre-incubated biological indicators arc placed to detect biological growth in the pre incubated biological indicators, wherein the comparison takes into account baseline fluorescence measurements obtained for reference biological indicators placed in the respective measurement wells.

2. The method of claim 1, further comprising:
obtaining the baseline fluorescence measurements for the reference biological indicators placed within a plurality of measurement wells; and
receiving the pre-incubated biological indicators within the measurement wells.

3. The method of claim 2, further comprising generating the threshold fluorescence values by summing predetermined threshold fluorescence values with the baseline fluorescence measurements to produce the threshold fluorescence values.

4. The method of claim 2, further comprising subtracting the baseline fluorescence measurements from the single fluorescence emission measurements for the respective measurement wells, and comparing the resulting differences to the respective threshold fluorescence values.

5. The method of claim 2, further comprising generating output indicating whether biological growth was detected in the pre-incubated biological indicators based on the comparison.

6. The method of claim 2, Further comprising:
placing a second set of the pre-incubated biological indicators within the measurement wells
obtaining single fluorescence emission measurement for each of the pre-incubated biological indicators in the second set; and
comparing the single fluorescence emission measurements obtained for cach of the pre-incubated biological indicators in the second set to the threshold fluorescence values for the respective measurement well to detect biological growth in the pre-incubated biological indicators in the second set.

7. The method of claim 2, wherein the biological indicators include biological indicators configured for ethylene oxide sterilization processes or for steam-based sterilization processes.

8. A device for performing the method of any of claims 1-7, the devices comprising:
a fluorescence emission reader to obtain fluorescence measurements for biological indicators placed within a plurality of measurement wells; and
a processor that controls the fluorescence reader to obtain a single fluorescence measurement for each of a plurality of pre-incubated biological indicators placed in the measurement wells, wherein the processor compares the single fluorescence emission measurements to threshold fluorescence values for the respective measurement wells in which the pre-incubated biological indicators are placed to detect biological growth in the pre-incubated biological indicators, wherein the comparison takes into account the baseline fluorescence measurements obtained for reference biological indicators placed in the respective measurement wells.

9. The device of claim 8, wherein:
the processor controls the fluorescence emission reader to obtain the baseline fluorescence measurements for each of the reference biological indicators placed within the measurement wells.

## Patentansprüche

1. Verfahren, umfassend:
Erhalten einer einzelnen Fluoreszenzmessung für jeden von mehreren vorinkubierten biologischen Indikatoren, die in Messschächten angeordnet sind; und
Vergleichen der einzelnen Fluoreszenzemissionsmessungen mit Fluoreszenzschwellenwerten für die jeweiligen Messschächte, in denen die vorinkubierten biologischen Indikatoren angeordnet sind, um das biologische Wachstum in den vorinkubierten biologischen Indikatoren zu erkennen, wobei der Vergleich Fluoreszenzgrundlinienmessungen berücksichtigt, die für biologische Referenzindikatoren erhalten werden, die in den jeweiligen Messschächten angeordnet sind.

2. Verfahren nach Anspruch 1, ferner umfassend:
Erhalten der Fluoreszenzgrundlinienmessungen für die biologischen Referenzindikatoren, die in mehreren Messschächten angeordnet sind; und
Aufnehmen der vorinkubierten biologischen Indikatoren in den Messschächten.

3. Verfahren nach Anspruch 2, ferner umfassend das Erzeugen der Fluoreszenzschwellenwerte durch Summieren der vorbestimmten Fluoreszenzschwellenwerte mit den Fluoreszenzgrundlinienmessungen, um die Fluoreszenzschwellenwerte zu erstellen.

4. Verfahren nach Anspruch 2, ferner umfassend das Subtrahieren der Fluoreszenzgrundlinienmessungen von den einzelnen Fluoreszenzemissionsmessungen für die jeweiligen Messschächte und Vergleichen der resultierenden Differenzen mit den jeweiligen Fluoreszenzschwellenwerten.

5. Verfahren nach Anspruch 2, ferner umfassend das Erzeugen einer Ausgabe, welche anzeigt, ob basierend auf dem Vergleich in den vorinkubierten biologischen Indikatoren ein biologisches Wachstum erkannt wurde.

6. Verfahren nach Anspruch 2, ferner umfassend:
Anordnen eines zweiten Satzes der vorinkubierten biologischen Indikatoren in den Messschächten;
Erhalten einer einzelnen Fluoreszenzemissionsmessung für jeden der vorinkubierten biologischen Inkubatoren in dem zweiten Satz; und
Vergleichen der einzelnen Fluoreszenzemissionsmessungen, die für jeden der vorinkubierten biologischen Indikatoren in dem zweiten Satz erhalten werden, mit den Fluoreszenzschwellenwerten für den jeweiligen Messschacht, um in den vorinkubierten biologischen Indikatoren in dem zweiten Satz ein biologisches Wachstum zu erkennen.

7. Verfahren nach Anspruch 2, wobei die biologischen Indikatoren biologische Indikatoren enthalten, die für Ethylenoxid-Sterilisierungsprozesse oder für auf Dampf basierende Sterilisierungsprozesse konfiguriert sind.

8. Vorrichtung zum Ausführen des Verfahrens nach einem der Ansprüche 1 bis 7, wobei die Vorrichtung umfasst:
ein Fluoreszenzemissionslesegerät, um Fluoreszenzmessungen für biologische Indikatoren zu erhalten, die in mehreren Messschächten angeordnet sind; und
einen Prozessor, der das Fluoreszenzlesegerät steuert, um eine einzelne Fluoreszenzmessung für jeden von mehreren vorinkubierten biologischen Indikatoren zu erhalten, die in den Messschächten angeordnet sind, wobei der Prozessor die einzelnen Fluoreszenzemissionsmessungen mit den Fluoreszenzschwellenwerten für die jeweiligen Messschächte vergleicht, in denen die vorinkubierten biologischen Indikatoren angeordnet sind, um das biologische Wachstum in den vorinkubierten biologischen Indikatoren zu erkennen, wobei der Vergleich die Fluoreszenzgrundlinienmessungen berücksichtigt, die für biologische Referenzindikatoren erhalten werden, die in den jeweiligen Messschächten angeordnet sind.

9. Vorrichtung nach Anspruch 8, wobei:
der Prozessor das Fluoreszenzemissionslesegerät steuert, um die Fluoreszenzbasislinienmessungen für jeden der biologischen Referenzindikatoren zu erhalten, die in den Messschächten angeordnet sind.

## Revendications

1. Méthode comprenant :
l'obtention d'une mesure unique de fluorescence pour chacun d'une pluralité d'indicateurs biologiques mis en incubation au préalable placés dans des alvéoles de mesure ; et
la comparaison des mesures uniques d'émission de fluorescence à des valeurs de fluorescence de seuil pour les alvéoles de mesure respectives dans lesquelles les indicateurs biologiques mis en incubation au préalable sont placés pour détecter une croissance biologique dans les indicateurs biologiques mis en incubation au préalable, dans laquelle la comparaison tient compte de mesures de fluorescence de base obtenues pour des indicateurs biologiques de référence placés dans les alvéoles de mesure respectives.

2. Méthode selon la revendication 1, comprenant en outre :
l'obtention des mesures de fluorescence de base pour les indicateurs biologiques de référence placés dans une pluralité d'alvéoles de mesure ; et
la réception des indicateurs biologiques mis en incubation au préalable dans les alvéoles de mesure.

3. Méthode selon la revendication 2, comprenant en outre la production des valeurs de fluorescence de seuil par calcul de la somme des valeurs de fluorescence de seuil prédéterminées et des mesures de fluorescence de base pour produire les valeurs de fluorescence de seuil.

4. Méthode selon la revendication 2, comprenant en outre la soustraction des mesures de fluorescence de base des mesures uniques d'émission de fluorescence pour les alvéoles de mesure respectives, et la comparaison des différences ainsi obtenues avec les valeurs de fluorescence de seuil respectives.

5. Méthode selon la revendication 2, comprenant en outre la production d'une sortie indiquant si une croissance biologique a été détectée ou non dans les indicateurs biologiques mis en incubation au préalable à partir de la comparaison.

6. Méthode selon la revendication 2, comprenant en outre :
la mise en place d'un deuxième ensemble d'indicateurs biologiques mis en incubation au préalable dans les alvéoles de mesure ;
l'obtention d'une mesure unique d'émission de fluorescence pour chacun des indicateurs biologiques mis en incubation au préalable dans le deuxième ensemble ; et
la comparaison des mesures uniques d'émission de fluorescence obtenues pour chacun des indicateurs biologiques mis en incubation au préalable dans le deuxième ensemble aux valeurs de fluorescence de seuil pour les alvéoles de mesure respectives pour détecter une croissance biologique dans les indicateurs biologiques mis en incubation au préalable dans le deuxième ensemble.

7. Méthode selon la revendication 2, dans laquelle les indicateurs biologiques comprennent des indicateurs biologiques configurés pour des procédés de stérilisation par l'oxyde d'éthylène ou pour des procédés de stérilisation par la vapeur d'eau.

8. Dispositif pour la mise en oeuvre de la méthode selon l'une quelconque des revendications 1-7, le dispositif comprenant :
un lecteur d'émission de fluorescence pour obtenir des mesures de fluorescence pour des indicateurs biologiques placés dans une pluralité d'alvéoles de mesure ; et
un processeur qui contrôle le lecteur de fluorescence pour obtenir une mesure unique de fluorescence pour chacun d'une pluralité d'indicateurs biologiques mis en incubation au préalable placés dans les alvéoles de mesure, dans lequel le processeur compare les mesures uniques d'émission de fluorescence à des valeurs de fluorescence de seuil pour les alvéoles de mesure respectives dans lesquelles les indicateurs biologiques mis en incubation au préalable sont placés pour détecter une croissance biologique dans les indicateurs biologiques mis en incubation au préalable, dans lequel la comparaison tient compte des mesures de fluorescence de base obtenues pour des indicateurs biologiques de référence placés dans les alvéoles de mesure respectives.

9. Dispositif selon la revendication 8, dans lequel :
le processeur contrôle le lecteur d'émission de fluorescence pour obtenir les mesures de fluorescence de base pour chacun des indicateurs biologiques de référence placés dans les alvéoles de mesure.
